(19) Europäisches Patentamt
European Patent Office
Office européen des brevets

(11) Publication number: **0 528 854 B1**

# (12) EUROPEAN PATENT SPECIFICATION

(45) Date of publication of patent specification: **28.12.94**

(51) Int. Cl.$^{5}$: **C07K 5/00**, C07K 7/00, C12Q 1/37

(21) Application number: **91908699.1**

(22) Date of filing: **16.04.91**

(86) International application number: **PCT/DK91/00102**

(87) International publication number: **WO 91/16336 (31.10.91 91/25)**

## (54) FLUOROGENIC PEPTIDES AND THEIR USE IN THE DETERMINATION OF ENZYMATIC ACTIVITIES.

(30) Priority: **17.04.90 DK 939/90**

(43) Date of publication of application: **03.03.93 Bulletin 93/09**

(45) Publication of the grant of the patent: **28.12.94 Bulletin 94/52**

(84) Designated Contracting States: **AT BE CH DE DK ES FR GB GR IT LI LU NL SE**

(56) References cited:
**EP-A- 0 240 914**
**US-A- 4 314 936**

**Chem. Pharm. Bull., Vol. 37, No.1, January 1989 Eisuke Sato et al.: "Novel Fluorogenic Substrates Containing Bimane System for Microdetermination of Angiotensin I Converting Enzyme", see page 145 - page 147**

**SCIENCE, Vol. 247, February 1990 Edmund D. Matayoshi et al: "Novel Fluorogenic Substrates for Assaying Retroviral Proteases by Resonance Energy Transfer", see page 954 - page 958**

(73) Proprietor: **CARLSBERG A/S**
**Vesterfaelledvej 100**
**DK-1799 Copenhagen V (DK)**

(72) Inventor: **MELDAL, Morten**
**Malov Hovedgade 109**
**DK-2760 Malov (DK)**
Inventor: **BREDDAM, Klaus**
**Sondervangsvej 28B**
**DK-2600 Glostrup (DK)**

(74) Representative: **Christiansen, Ejvind et al**
**HOFMAN-BANG & BOUTARD A/S**
**Adelgade 15**
**DK-1304 Copenhagen K (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to fluorogenic peptides. More particularly it relates to peptides exhibiting the so-called intramolecular or internal quenching of the fluorescence brought about by a fluorescent chromophore (donor group) by another chromophore (acceptor group). Such peptides are particularly useful in the determination of enzymatic specificities since enzymatic cleavage of a peptide bond between the chromophore groups will result in an increase in fluorescence. Therefore a series of internally quenched fluorogenic peptides of varying amino acid sequence and chain length would be a valuable tool e.g.in screening programs for the determination of the enzymatic activities of various biological systems. Such assays could facilitate the study of biological proteases. e.g. derived from HIV-1 and cellular proteases.

Description of the prior art

In the present description the usual abbreviations within the peptide chemistry are employed. The amino acid symbols are in accordance with IUPAC-IUB Commission on Biochemical Nomenclature, and the amino acids are in L-form unless otherwise stipulated. The one letter code for the amino acids used in table 1 is as recommended by IUPAC, Eur. J. Biochem. 138, p. 37 (1984). The following additional abbreviations are used:

| | |
|---|---|
| ABz | = o-aminobenzoyl |
| HPLC | = High performance liquid chromatography |
| PNa | = p-nitroanilide |
| Np | = p-nitrophenyl |
| AEAP | = aminoethyl-2-aminopyridine |
| CBI | = cyanobenzoisoindole |
| dansyl, dns | = 5-dimethylaminonaphthylsulphonyl |
| Bim | = bimane |
| Boc | = t-butyloxycarbonyl |
| DMF | = N,N-dimethylformamide |
| Dhbt- | = 3,4-dihydro-4-oxo-1,2,3-benzotriazo-3-yl |
| DCCI | = N,N'-dicyclohexylcarbodiimide |
| Fmoc | = fluoren-9-ylmethyloxycarbonyl |
| NSu | = N-succinimidyl |
| IR | = infrared spectroscopy |
| THF | = tetrahydrofurane |
| TFA | = trifluoroacetic acid |
| Tlc | - thin layer chromatography |
| EtOAc | = ethylacetate |
| tBu | = t-butyl |
| TBTU | = N,N'-tetramethyl-O-benzotriazo-1-yluronium tetrafluoroborate |

In the following the nomenclature (..$S_2$,$S_1$,$S_1$',$S_2$'...) has been used for defining the subsite-specificity of the enzymes, and analogously (..$P_2$,$P_1$,$P_1$',$P_2$'...) for the corresponding binding position of the substrates in these sites, as stated by Schechter and Berger in Biochem. Biophys. Res. Comm. vol. 27, p. 157-162 (1967)

Proteolytic enzymes usually exhibit a preference for certain peptide bonds in a polypeptide-chain. With some enzymes. e.g. trypsin, the rate of hydrolysis is primarily determined by the nature of the amino acid residues forming the peptide bond, suggesting that the primary binding sites, i.e. $S_1$ and $S_1$', are the most important for the interaction between the enzyme and the substrate. With other enzymes the nature of the amino acids further removed from the scissile bond, interacting with the so-called secondary binding sites, exert a similar or even greater influence on the rate of hydrolysis. In extreme cases, e.g. some of the enzymes involved in hormone processing and blood clotting, cleavage will only take place when the enzyme recognises a defined amino acid sequence around the scissile bond. However, even for enzymes generally accepted to be non-specific such as the subtilisins secondary interactions are known to play a dominant role as well. To understand how such interactions, remote from the catalytic event, influence cleavage rates and to evaluate the consequences of site directed mutagenesis at the binding site, it is necessary to map the preferences of the enzyme with respect to each position of the substrate in a much more detailed way than previously performed. Such studies have furthermore become topical due to the application of proteolytic enzymes for specific cleavage of fusion proteins produced by genetically engineered microorganisms. The utilization of such enzymes for synthesis of peptide bonds, generating

highly pure biologically active peptides would benefit from such knowledge as well.

Information on protease specificity has been obtained using synthetic peptides, four to eight amino acid residues of length, and following the hydrolysis by HPLC or more recently, by proton NMR-spectroscopy (Meldal, M & Breddam, K. the Proceedings of the solid Phase Synthesis Symposium, Oxford 1989, in press). However, such methods are slow, laborious and insensitive, and it is therefore required that the synthetic peptides are suitably derivatised with chromophores such that their hydrolysis can be followed by a change in absorbance or fluorescence.

The spectrophotometric determination of the release of p-nitroaniline or p-nitrophenol from the C-terminus of peptide-PNa and peptide-ONp substrates, respectively, are among the most commonly employed methods. Unfortunately, these methods are not very sensitive, and the scissile bonds are not natural peptide bonds. Furthermore, only the influence of the substrate positions $P_1$, $P_2$, .. $P_n$ and not the positions $P_1$, $P_2'$, ... $P_n'$ may be studied (c.f. fig. 1). In addition, substrates of this type are base labile and can only be synthesized by elaborate synthesis in solution. Another type of substrate involves the hydrolysis of the -Phe($NO_2$)-Phe- peptide bond which may be followed by spectrophotometric detection of the change in nitrophenyl absorbance. This method exhibits very low sensitivity and requires that the -Phe-($NO_2$)- residue is accepted in the $P_1$ subsite. More sensitive substrates can be obtained by incorporation of fluorescent probes. Such peptide substrates have been constructed by attachment of fluorescent groups at the C- or the N-terminus of the peptides. Peptide β-naphthyl amides are frequently used as sensitive fluorescent substrates. Aminoethyl-2-aminopyridine (AEAP) has been used for C-terminal fluorescence labeling. Other probes for N-terminal labeling are N-pyridin-2-yl glycine and the cyanobenzoisoindole (CBI). The dansyl group (1-dimethylaminonaphthalene-5-sulfonyl) is normally attached to the N-terminal, but solid phase synthesis of peptides have been reported in which the dansyl group is attached either to a C-terminal ethylene diamine after cleavage from the resin or to the side chain of a lysine residue. Dansyl substrates have been applied to the study of enzyme substrate complexes. Since dansylated (and mansylated) peptides change the intensity of their fluorescence depending on the polarity of the micro environment an increase in fluorescence is observed as the substrate is transferred from water to the more hydrophobic enzyme cavity. The proximity of the dansyl group to tryptophan residues in the enzyme results in energy transfer from the tryptophan to the dansyl group, cf. Yaron et al. (1979) Anal. Biochem. 95, 228-235.

The intramolecularly quenched substrates constitute another group of fluorescent substrates. These substrates in addition to the fluorescent group contain a quenching chromophore, and when a peptide bond situated between these is cleaved by enzymes an increase in fluorescence is observed. As described by Yaron et. al., op. cit. and US patent No. 4.314.936, both incorporated by reference, two mechanisms are operating in the energy transfer process for internal quenching. Quenching by intramolecular collision requires a short distance between the fluorescent donor and the acceptor so that the necessary formation of a contact complex occurs frequently enough to ensure effective transfer. By contrast, the quenching of fluorescence by long range resonance energy transfer is effective over long distances. Thus transfer from fluorescein to chlorophyll can occur at distances of 80 Å in solution. The consequence of the resonance energy transfer theory, as described by Förster (1948) Ann. Physik 6 (2), 55-75, is that the transfer process is a non radiative parallel process in which the donor returns to its ground state simultaneously with the excitation of the acceptor. The efficiency of the transfer is directly related to the spectral integral overlap of the emission of the donor chromophore with the absorption of the acceptor chromophore. The transfer decreases with the sixth power of the distance between the chromophores and thus, the spectral overlap must be complete to ensure effective quenching.

Of the internally quenched substrates described in the literature most are of the collision type and only a few amino acids can be inserted between the donor and the acceptor. The anthraniloyl (2-aminobenzoyl or ABz) group, which is a small fluorescent probe with a high quantum yield, has been used in conjunction with the -Phe($NO_2$)-group, see e.g. US patent No. 4.314.936 and Yaron et al op. cit., but these substrates are of the collision type as the -Phe($NO_2$)- group only functions as quencher when separated by no more than two residues. The dansyl group has also been used for the collision type intramolecularly quenched substrates in combination with -Phe($NO_2$)-, Fleminger et al. (1981) FEBS Letters 135, 131-134. Other fluorescent probes used for internally quenched collision type substrates are the bimane group (Bim), Sato et al. (1988) Bioorganic Chem. 16, 298-306, and the naphthyl group, Yaron et al. op. cit.. The Bim group was incorporated into short peptides containing a Trp residue which quench the Bim fluorescence up to the tripeptide level. Peptide 2-naphthylethylamines derivatized at the N-terminal with a anthracen-9-ylcarbonyl group quenching the naphthyl fluorescence has been used for trypsin substrates, cf. Yaron et al.

Even the tryptophan containing dansyl peptides, which are considered to be of the resonance energy transfer type, has only a 4.5 fold quenching for the tetrapeptide Dns-gly-gly-Gly-Trp and the quenching is decreased with a factor of 5 for each addition of a residue, cf. Yaron et al.

The heptapeptide Dns-Gly-Lys-Tyr-Ala-Pro-Trp-Val has been used to explore the protease specificity of a number of enzymes, Ng & Auld, (1989) Anal. Biochem. 183, 50-56.

This peptide exhibits intramolecular quenching of the tryptophan fluorescence by the dansyl group, which are separated from Trp by a hexapeptide sequence.

The straight chain distance between the Dansyl group and the Trp is about 22 Å, which should only lead to a 50% quenching. However, the results showed that quenching was more than 85% efficient, indicating that the two chromophores may be closer than straight chain calculations predict or fixed in a conformation that is optimal for energy transfer, possibly due to the presence of an intermediate Pro-residue.

The abovementioned peptide thus represents a special case, and it is fair to say that the Dns/Trp combination is not generally applicable to internal quenching of longer peptides.

Otherwise, the dansyl containing substrates would be ideal since they can be synthesized on a solid phase and can be prepared by parallel multiple column peptide synthesis, which is compulsory to obtain the large variety of peptides necessary in a screening program on new mutant enzymes. Multiple column peptide synthesis is described by Holm, A & Meldal, M (1988) Peptides 1988, Proc. 20'th Eur. Pept. Symp. 208-210 and in application WO 90/02605 both, incorporated by reference. Spectroscopically more promising and of the long range energy transfer typs is the combination of the anthraniloyl group and the PNa-group, Bratavanova, E. K. & Petkov, Q. O. (1987) Biochem. J. 248, 957-960 and (1987) Anal. Biochem. 162, 213-218, allowing a separation of at least four residues. However, such substrates cannot be prepared by conventional solid phase or multiple column peptide synthesis, and the C-terminal peptide bond is unstable.

Matayoshi et al., Science, (1990) 247, 954-958), investigated internally quenched fluorogenic substrates based on the DABCYL/EDANS pair.

They investigated two octapeptides

Ser-Gln-Asn-Tyr-Pro-Ile-Val-Gln and

Ser-Val-Val-Tyr-Pro-Val-Val-Gln

having as the C-terminal fluorescent donor EDANS (5-(2-aminoethyl/amino)naphthalene-1-sulphonic acid and as the quenching acceptor DABCYL (4-(4-dimethylaminophenylazo)benzoic acid linked via a GABA ($\gamma$-aminobutyric acid) spacer.

The octapeptides are derived from HIV-1PR, an 11kd protease encoded by HIV-1 virus, and the related protease derived from avian myeloblastosis virus.

The bulky nature of the DABCYL group necessitates the use of a GABA or other spacer to avoid potential steric hindrance of substrate binding.

Also these type of substrates cannot be prepared by multiple column peptide synthesis due to the nature of the EDANS group.

EP-A-435845 published July 3, 1991 is prior art under Art. 54(3) for all the designated contracting states. It describes certain fluorogenic hexapeptides exhibiting intramolecular quenching of the collision type.

A representative example is

ABz-Thr-Ile-Nle-Phe(p-$NO_2$)-Gln-Arg-$NH_2$

wherein ABz is the fluorogenic donor and Phe(p-$NO_2$) is a collision type quenching acceptor. Modified Tyr as defined in claim 1 is not disclosed as quencher.

## Object of the invention

The object of the present invention is to provide a class of intramolecularly quenched fluorogenic peptides which are not entailed with the abovementioned drawbacks.

More specifically it is the object of the invention to provide a large variety of peptides which are intramolecularly quenched by long range resonance energy transfer and where the quenching is effective over distances of more than 50 Å, i.e. where the donor and acceptor is separated by up to more than 15 amino acids.

A further object of the invention is to provide peptides having the above characteristics, which lend themselves to synthesis by simultaneous parallel multiple column synthesis.

## Summary of the invention

The invention is based on the surprising finding that these objects can be achieved by selection of a special donor/acceptor pair, viz. by using the anthraniloyl (o-aminobenzoyl) group as the fluorescent donor and 3-nitrotyrosine as the quenching acceptor.

The anthraniloyl group is a small, polar group which has a high quantum yield. It enhances the solubility of the peptide in water as compared to other more lipophilic fluorescent probes, and can be excited at 320 nm, well outside the spectral region of the enzymes and the peptide amino acids. The anthranilamide emission exhibits its maximum at 420 nm, far from the excitation wavelength. The quencher must therefore absorb in the vicinity of 420 nm with an absorption band of the same width and shape as the emission band of the anthranilamide. Furthermore, it is preferable that it contains a carboxylic acid for attachment to the solid phase and an amino group for amide bond formation with the peptide. It was found that 3-nitrotyrosine fulfills these requirements, and that it can easily be incorporated into the Fmoc based solid phase peptide synthesis by the continuous flow polyamide method.

3-nitrotyrosine has not earlier been described as a fluorescence quencher, but only for use in the spectroscopical characterization of the position of tyrosine in proteins by derivatization with tetranitromethane, Riordan et al. (1967) Biochemistry 6, 358-361. Due to its absorption band it can be used not only with anthraniloyl but also with other fluorophores having an emission in the spectral region of 400-460 nm, e.g. 1,8-aminonaphthalene sulfonic acid, anthracene and umbelliferone. By the same token, compounds structurally related to 3-nitrotyrosine may also be used as the quenching acceptors, the only condition being that a fluorescent donor should be used which has a maximum emission within the absorption band for the quenching acceptor, so that the spectral overlap is as complete as possible.

Accordingly, the present invention relates to fluorogenic peptides exhibiting intramolecular quenching by resonance energy transfer and having the general formula

ABz-A-Q-$Y_1$ or $Y_2$-Q-A-ABz

wherein ABz designates the anthraniloyl donor chromophore and Q is a fluorescence quenching acceptor chromophore having an absorption spectrum overlapping the emission spectrum of the ABz donor chromophore, being a substituted amino acid residue of the general formula

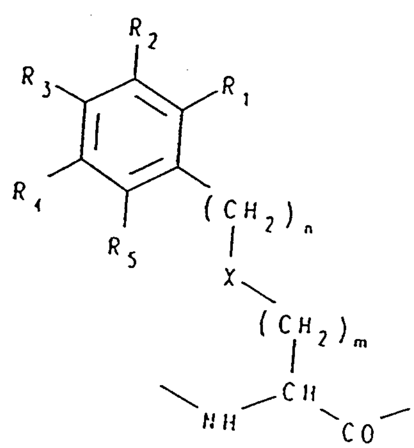

wherein $R_1$, $R_2$, $R_3$, $R_4$ and $R_5$ are independently selected from hydrogen, hydroxy, alkyl, alkoxy, nitro, amido, halogen or aralkyl, with the proviso that at least one of $R_1$ to $R_5$ is hydroxy and at least one is nitro, wherein X is a spacing group which may be absent or selected from any combination of -CO-NH-, -CO-O-, -S-, -S-S-, -O-, -NH- or -$C_6H_4$-, and n and m are integers from 0 to 10,
A is a spacing entity selected from amino acid residues and peptide residues and may include a non-peptide molecular entity, being linked to the fluorescent donor chromophore ABz via an amide bond or ester bond,
$Y_1$ is OH, $NH_2$, an amino acid residue, a peptide residue or a C-terminal protective group,
$Y_2$ is H, an amino acid residue, a peptide residue or an N-terminal protective group.

The preferred quenching acceptor chromophore Q is 3-nitrotyrosine, i.e. n = 1, $R_3$ = OH and $R_4$ = $NO_2$. Accordingly a preferred group of fluorogenic peptides is that having the general formula

ABz-$A_1$-Tyr($NO_2$)-$Y_1$

wherein $A_1$ is an amino acid residue or a peptide residue linked to the ABz group via a peptide bond, and $Y_1$ is as defined above.

As mentioned, A is an amino acid residue or preferably a peptide residue, in particular a peptide residue consisting of from 2 to 25, preferably 2 to 15 amino acids.

For the sake of clarity it should be noted that the symbols A, Q and Y used in the above formulae should not be confused with the one letter codes for amino acids used below in Table 1.

In the present context "alkyl" means straight chain or branched alkyl, preferably with 1 to 6 carbon atoms, e.g. methyl, ethyl, propyl, isopropyl, n-butyl, isobutyl, tert.butyl, amyl and hexyl.

"Alkoxy" is defined analogously.

"Aryl" e.g. encompasses heterocyclic aryl and e.g. phenyl.

"Aralkyl" e.g. encompasses benzyl and phenethyl.

The above-mentioned groups may optionally be substituted with inert substituent, e.g. halogen or nitro.

"Halogen" denominates F, Cl, Br and I.

The character of the spacing amino acids are not critical and may include non-amino acid entities, e.g. mono- or oligosaccharides.

Examples of useful amino acids include aliphatic amino acids, such as monoaminomonocarboxylic acids, e.g. glycine (Gly), alanine (Ala), valine (Val), norvaline (Nval), leucine (Leu), isoleucine (iso-Leu) and norleucine (Nleu), hydroxy amino acids, such as serine (Ser), threonine (Thr) and homoserine (homo-Ser), sulfur-containing amino acids, such as methionine (Met) or cystine (CysS) and cysteine (CysH), monoaminodicarboxylic acids, such as aspartic acid (Asp), glutamic acid (Glu) and amides thereof, such as asparagine (Asn) and glutamine (Gln), diaminomonocarboxylic acids, such as ornithine (Orn) and lysine (Lys), arginine (Arg), aromatic amino acids, such as phenylalanine (Phe) and tyrosine (Tyr), as well as heterocyclic amino acids, such as histidine (His), proline (Pro) and tryptophan (Trp). As examples of useful amino compounds of a more unusual structure may be mentioned penicillamine (Pen), aminophosphonic acids, such as alanine-phosphonic acid (AlaP), aminosulfonic acids, such as taurine (Tau), $\omega$-amino acids, such as $\beta$-alanine (BAla), iso amino acids, such as $\alpha$-methylalanin (Aib), amino acids substituted with inert substituents, e.g. halogen or nitro. They may be included both in D-form where applicable and in L-form.

If desired, the individual amino acids may be side-chain protected in a manner known per se.

Since the peptides according to the invention are particularly valuable for purposes of screening new enzymes or enzyme containing fluids for their activities, it might be desirable to incorporate more special compounds in the peptide chain.

Generally speaking the expression "peptide residue" should be construed broadly as not only covering sequences of amino acids solely linked by peptide bonds, but also encompassing sequences where one or more of the bonds are not peptide bonds, but also e.g. ester bonds such as found in the socalled depsipeptides. The spacing entity may also include non-peptide entities, e.g. mono- or oligosaccharide fragments, e.g $(\alpha\text{-D-Glc}(1\text{-}4))_4$.

In particular the anthraniloyl group ABz need not necessarily be bound to an amino acid via an amide or peptide bond.

Depending on the character of the amino acid to which it is attached, ABz may also be bound e.g. via an ester bond.

Thus, in case of the N-terminal amino acid being a hydroxyamino acid like Ser and Thr, both an ester bond and an amide bond is possible.

Likewise, in case of amino acids containing more than one amino group, the antraniloyl group need not be attached to the $\alpha$-amino group.

Thus, e.g. in case of the C- or N-terminal amino acid being Lys, the ABz group may well be attached to the $\epsilon$-amino group.

The invention also relates to a substrate for determination of enzymatic activities comprising one or more fluorogenic peptides as defined above.

In a still further aspect the invention relates to a process for the detection or determination of enzymatic activities in a sample, wherein the sample is incubated with one or more substrates as defined above under conditions favourable to enzymatic activities, i.a. pH 2-12, determining and comparing the fluorescence emitted before and after the incubation.

The syntheses of the protected precursors of the preferred chromophores ABz and Tyr($NO_2$) are outlined in the reaction scheme (Fig. 2) and explained in more detail below. The acylation with anthranilic acid requires a high level of activation to ensure complete coupling. It is not sufficient to leave the amino group of anthranilic acid unprotected and activate the carboxyl group as the benzotriazole ester as for synthesis in solution since such compounds exhibit a low reactivity. This may be ascribed to interaction of the unprotected amino group and the carboxyl group, and protection of the amino group should therefore enhance the reactivity. Such a protection has the additional advantage of preventing the incorporation of two or more anthranilic acid residues. Thus anthranilic acid (1) was reacted with $Boc_2O$ in dry DMF and

triethylamine to yield 60% of Boc-ABz-OH (2) and this was transformed into the highly activated Boc-ABz-O-Dhbt (3) in a 97% yield by reaction with DCCI and Dhbt-OH using the procedure of Atherton et. al. (1988) J. Chem. Soc. Perkin Trans 1, 2887-2894. Compound 3 could be used for fast coupling reactions on the solid phase.

3-nitrotyrosine (4) could be converted into Fmoc-Tyr($NO_2$)-OH (5) in a 91% yield by reaction with Fmoc-O-NSu under the Schotten-Baumann conditions. The phenolic hydroxyl group need no protection since any acylation of the activated phenol can be reverted by treatment with weak bases e.g. piperidine. For solid phase synthetic purposes compound 5 can be coupled directly to the hydroxymethyl group of resin linkers by reaction with 0.5 eqv. of DCCI and DMAP catalysts. Alternatively, one or more amino acids can be coupled to the resin prior to the reaction with Fmoc-Tyr-($NO_2$)-OH promoted by the TBTU reagent of Knoor.

In order to show the versatility of the system the peptides 6-15 shown in table 2 were prepared by parallel multiple column peptide synthesis in a 20 well manual synthesizer, cf. Meldal & Breddam, op. cit. and Holm & Meldal, op. cit.. The peptides were synthesized by the Dhbt ester method described by Atherton et. al., op. cit.. As further explained below, the completion of acylations were monitored visually to ensure high purity of all the peptide products, see also Cameron et al. J. Chem. Soc. Perkin Trans 1, 2895-2901, incorporated by reference. The peptides were purified by preparative HPLC, and the pure peptides were used for kinetic experiments. The endopeptidase subtilisin A was selected as the test enzyme.

The synthesized substrates were selected on the basis of existing knowledge of the substrate preference of subtilisin A: a) preference for an aromatic amino acid residue in the $P_1$ position, b) preference for a non-bulky residue in the $P_1'$ position, c) the presumed ability of the enzyme to accomodate the -Tyr-($NO_2$)- at the $P_2'$ and finally d) the enormous influence of interactions between the substrate and the $S_2$, $S_3$ and $S_4$ subsites, see Philipp & Bender, (1983) Mol. Cell. Biochem. 51, 5-32, i.e. the influence of chainlength on the rate of hydrolysis. Aspartic acid was chosen as the C-terminal to enhance the solubility and to protect the peptides against digestion by adventitious carboxypeptidases. On this basis it was predicted that the -Phe-Gly- or -Phe-Ser- bonds were the preferred cleavage points in all the synthetized substrates. The cleavage points of the long peptides were determined by amino acid analysis of the products after separation by HPLC, and the -Phe-Gly- cleavage point was confirmed.

## Brief description of the drawings

Fig. 1 illustrates the subsite specificity for an enzyme ($S_3$, $S_2$, $S_1$, $S_1'$, $S_2'$) with regard to a scissile bond on a peptide substrate and the corresponding binding positions of the substrate ($P_3$, $P_2$, $P_1$, $P_1'$, $P_2'$).

Fig. 2 illustrates the synthesis of precursors for the preferred fluorescent and quenching chromophores.

Fig. 3 shows the enzymatic hydrolysis of a peptide according to the invention.

## Detailed description of the preferred embodiments

## EXPERIMENTAL PROCEDURES

General procedures. Solvents were distilled at the appropriate pressure in a packed column of Raschig rings. DMF was analyzed by mixing with Dhbt-OH prior to use. THF and dioxane were passed over a short column of active alumina for purification and desication. Fmoc amino acids were purchased from Bachem or Milligen and were transformed into active Dhbt esters as described by Atherton et al. op. cit. The purity of the Dhbt esters was checked by HPLC and IR spectroscopy. Fmoc-O-NSu, nitrotyrosine, Dhbt-OH, DCCI and $Boc_2O$ were from Fluka. "Macrosorb-SPR 250" was purchased from Sterling Organics and was derivatised with ethylene diamine and hydroxymethylphenoxyacetic acid Dhbt ester. $^1$H-MNR-spectra were recorded on a Brucker AM500 500 MHz spectrometer, IR-spectra were recorded on a Perkin Elmer 157 spectrophotometer, HPLC was performed on a waters gradient HPLC system with a Novapak 5 $\mu$ $C_{18}$ reversed phase column with a buffer A (0.1 % TFA) and a buffer B (0.1 % TFA and 10% water in acetonitrile) with a flow rate of 1 ml/min and the appropriate gradient. Completely deprotected peptides were hydrolysed in 6 N HCl at 110° and analysed on a Durrum amino acid analyzer.

EXAMPLE 1

Preparation of the precursors for the preferred fluorescent and quenching chromophores (see Fig. 2)

2-t-Butyloxycarbonylamino benzoate (2). $Boc_2$ (51.6 g, 236 mmol), anthranilic acid (1) (25.9 g, 189 mmol) and triethylamine (50 ml, 360 mmol) were dissolved in DMF (50 ml). Within a few minutes gas evolution commenced and the mixture was left for a perriod of 24 h at 20° until the gas evolution had ceased. Tlc in EtOAc showed the reaction to be complete and the mixture was treated with charcoal, filtered through celite which was rinsed with DMF. The DMF was removed at 30° in vacuo, and the residue was dissolved in dichloromethane (200 ml) and extracted with 10% sodium carbonate solution (100 ml). The dark brown aqueous phase was extracted with dichloromethane and discarded. The combined organic phase was extracted with 3 times 100 ml of water.

The aqueous extractions were acidified to pH 2 and extracted with diethyl ether (3 x 100 ml). After drying with sodium sulfate and filtration the volume was reduced to 100 ml and petroleum ether was added until the product crystallized. The crude material was recrystallized from 50% aqueous ethanol to give 26 g (60%) of pure Boc-anthranilic acid (2), m.p. 149-150°. Anal. calc. for. $C_{12}H_{15}NO_4$: C 60.75, H 6.37, N 5.90%. Found: C 60.72, H 6.37, N 6.02%. $^1$H-NMR ($CDCl_3$): δ ppm (J Hz); Boc, 1.54; H3, 8.11 (7.7); H4, 7.57 (7.7, 7.5); H5, 7.04 (7.5, 8.4); H6, 8.48 (8.4); Boc, 1.55; COOH, 10.02.

3,4-Dihydro-4-oxo-1,2,3-benzotriazol-3-yl-2-t-butyloxycarbonylamino benzoate (3). 2-t-Butyloxycarbonylaminobenzoic acid (2) (2.37 g, 10 mmol) was dissolved in dichloromethane (15 ml) and THF (5 ml). The mixture was cooled to -5° and DCCI (2.06 g, 10 mmol) was added. After 5 min Dhbt-OH (10 mmol, 1.63 g) was added and the mixture was stirred at -5° for 1 h and at 4° for 16 h. After filtration the solvents were removed in vacuo, and the product was crystallized from diethyl ether (30 ml). Filtration afforded 3.7 g (97%) product which was pure according to HPLC. M.p. 155-156°. Anal. calc for $C_{19}H_{18}N_4O_5$: C 59.68, H 4.75, N 14.65%. Found: C 59.55, H 4.83, N 14.59. $^1$H-NMR ($CDCl_3$): δ ppm (J Hz); H3, 8.48 (7.7); H4, 7.72 (7.7, 7.4); H5, 7.19 (7.4, 7.8); H6, 8.61 (7.8); H5', 8.32 (7.4); H6', 8.10 (7.4, 7.7), 1.2); H7', 7.93 (7.7, 7.8); H8', 8.42 (7.8, 1.2); Boc, 1.52; NH, 9.58.

$N^\alpha$-Fluoren-9-ylmethyloxycarbonyl-3-nitrotyrosine (5). H-Tyr($NO_2$)-OH (4) (3.39 g, 15 mmol) was dissolved in 50 ml of water containing sodium carbonate (3.98 g, 38 mmol) and dioxane (20 ml) was added. Fmoc-O-NSu (5.20 g, 15.5 mmol) was dissolved in dioxane (20 ml) and added dropwise at 0°. The mixture was stirred 1 h at 0° and 3 h at 20°. The dioxane was removed in vacuo, and the residue diluted to 50 ml with water. Byproducts were extracted with ether, and the solution was acidified with citric acid. The precipitate was collected by filtration and dried. The product was extracted with ethyl acetate, and after filtration it was crystallized by addition of 3 volumes petroleum ether (2:7) and cooling. Collection of the crystalline material by filtration and washing with petroleum afforded 6.09 g of product (91% yield). M.p. 145-148. Anal. calc for $C_{19}H_{18}N_4O_5$: C 64.28, H 4.50, N 6.25. Found: C 63.44, H 4.58, N 6.36. $^1$H-NMR ($CDCl_3$): δ ppm (J Hz); 01-H, 10.52; H2, 4.72 (5.0, 6.0, 7.0 Hz); H3, 3.12 (13.5, 6.0); H3', 3.26 (13.5, 5.0); H5, 7.96; H5', 7.35 (8.0); H6', 7.12 (8.0); Fmoc 4.24 (6.5); 4.45 (6.5, 10.5); 4.54 (6.5, 10.5); 7.37 (7.0); 7.44 (7.5, 7.0); 7.54 (7.5, 7.5); 7.81 (7.5).

EXAMPLE 2

Multiple column synthesis of ABz-peptide-Tyr($NO_2$)-Asp on a 20 well synthesizer.

Fmoc-Asp(tBu)-OH (650 mg, 1.625 mmol) was dissolved in dichloromethane (15 ml) and the solution was cooled to 0°. DCCI (167 mg, 0.812 mmol) was added and the mixture was stirred for 15 min at 0°. After filtration and evaporation the residue was dissolved in DMF (5 ml) and added to a preswollen acid labile "Macrosorb SPR-250" resin (650 mg, 0.163 mmol) in a sintered funnel. DMAP (20.5 mg, 0.163 mmol) was added, and the material was shaken by a rotational motion for 1 h. The resin was washed with DMF (20 ml), and piperidine (20% in DMF, 15 ml) was added for deprotection purposes. After 10 min of shaking the piperidine was removed and the resin was washed with 100 ml DMF by the run through mode. Fmoc-Tyr-($NO_2$)-OH (219 mg, 489 mmol) and TBTU (157 mg, 489 mmol) was added and dissolved in DMF (15 ml). Diisopropyl ethyl amine (0.63 mg, 489 mmol) was added and the mixture was shaken for a period of 2 h. All reagent was removed by washing with DMF, and the DMF was removed with diethyl ether.

The resin was dried and measured in 90 mg quantities into the 7 wells of the 20 well manual multiple column peptide synthesizer. All solvents were handled with 100 ml polypropylene syringes. After swelling of the resin with DMF the solvent was drained from the resin by vacuum on the chamber beneath the wells, and a slight overpressure of air was established via a flowmeter. The Fmoc group was removed with a 1

min and a 10 min treatment with piperidine (20%) in DMF (500 µl in each well). The piperidine solution was removed by suction, and the wells were washed with DMF (5 times 1 ml each). The Fmoc amino acids Dhbt esters (2.5 eqv.) corresponding to position 3 from the C- terminal end of peptides 6-15 in table 1 below were weighed directly into the respective well in the synthesizer through a hole in a polyethylene cover hindering cross contamination. (In Table 1 the one letter code for the amino acids are used for the sake of simplicity). A slight overpressure was established and DMF (400 µl) was added to each well. For a period of 2 h the well system was shaken every 30 min. The reaction mixture was removed and the wells were washed with DMF (5 times 1 ml each). The synthesis cycle was repeated until the end of each peptide, 6-15 by application of the respective Fmoc amino acid Dhbt ester (2.5 eqv.).

The last residue added was Boc-ABz-O-DHBT (2.5 eqv.), and after a wash with DMF and diethyl ether the resins were dried. The peptides could directly be cleaved off the resins with aqueous TFA (95%, 1 ml), whereby also the Boc-group was removed from the ABz-group.

EXAMPLE 3

Enzymatic hydrolysis of intramolecularly quenched substrates. The peptides 6-15 prepared in example 2 were dissolved in DMF at a concentration of 0.2 - 1.0 mM. This solution (125 µl) was added to 50 mM Bicine, 2 mM $CaCl_2$, pH 8.5 (2375 µl), and the initial fluorescence of the solution at 420 nm by excitation at 320 nm was measured. From a stock solution of subtilisin A in the same Bicine buffer the appropriate amount was added to give final enzyme concentrations of $2*10^{-5}$ mg/ml - 2 mg/ml. The reactions were followed with time by monitoring the emission at 420 nm upon excitation at 320 nm. The temperature was maintained at 24 °C. The hydrolysis was carried out to completion and the specificity constants, $k_{cat}/K_m$, were determined from the progression curve using the integrated form of the Michaelis-Menten equation as described by Segel (1968) Biochemical Calculations, John Wiley & Sons 393-396.

When the peptides were dissolved in a 0.4-50 µm concentration only a limited fluorescence could be observed at 420 nm by excitation at 320 nm. With the addition of nM amounts of subtilisin A the fluorescence increased as exemplified for compound 6 in figure 3. For all the substrates having a chain length below 10, the fluorescence of the substrate was < 3% of the final cleavage product. For the longer peptides a gradual increase of the initial fluorescence was observed. Assuming an average length of each amino acid of 3 Å, the described donor acceptor system is effective over distances of more than 50 Å, and the anthranilamide and 3-nitrotyrosine can be considered an ideal long range resonance energy transfer pair.

The $k_{cat}/K_m$ values (table 1) for the hydrolysis of compound 6, 7 and 8 confirm the importance of chain length, as an elongation by one or two alanyl residues causes an increase in $k_{cat}/K_m$ of two and five orders of magnitude, respectively. Elongation by another two alanyl residues, i.e. compound 10, has only minor additional influence on $k_{cat}/K_m$.

However, with the three alanyl residues in compound 9, $k_{cat}/K_m$ is three to four fold lower than for compounds 8 and 10. This is somewhat surprising, and it indicates an influence of the ABz group increasing the preference for compound 8 or decreasing it for compound 9. The former possibility is suggested since the aromatic ABz group in this substrate is located in the $P_4$ position, and subtilisin A is known to exhibit a preference for aromatic amino acid residues in this position.

A comparison of the $k_{cat}/K_m$ values for the hydrolysis of compound 8 and 11 and of compound 8 and 12 shows that subtilisin A exhibits equal preference for Gly and Ser in the $P_1'$ position, and that Pro in the $P_2$ position has an adverse influence relative to Ala.

For compounds 13 to 15 the chain length is further increased in relation to compound 10 by each 3 amino acid residues. It is noteworthy that the $k_{cat}/K_m$ stabilizes at about 200.000, and decreases slightly for the very long substrates, while the efficiency of the intramolecular quenching diminishes with increasing chain length between the anthraniloyl group and the 3-nitrotyrosine residue.

Thus, for compound 15 where the distance is 16 amino acids corresponding to about 48 Å, an initial fluorescence of about 15% of the fluorescence after subtilisin cleavage is observed.

For practical screening purposes an upper limit of about 60-80 Å seems reasonable, corresponding to about 20-27 amino acids.

However, there is no well-defined upper limit for the number of intermediate amino acids. The above examples were deliberately chosen to make a comparison possible, and the amino acid sequences were designed to reduce the influence of the secondary and tertiary structure of the peptides.

In case of longer peptides e.g. having an α-helical structure intramolecular quenching may be obtained even if the donor/acceptor pair is separated by a greater number of amino acids as compared to an unordered structure.

EXAMPLE 4

Hydrolysis under acidic conditions

In order to further show the versatility of the fluorogenic peptides according to the invention the peptide ABz-Gly-Ala-Ala-Phe-Phe-Tyr($NO_2$)-Asp-OH (0.5$\mu$m) prepared as in example 2 was dissolved in 2.5 ml 50 mM formic acid, pH 3.4. The peptide was hydrolyzed by addition of pepsin (5$\mu$l, 0.1 mg/ml) yielding a progress curve with a 10 fold increase in fluorescence following hydrolysis for 1 1/2 hour.

For convenience the one-letter codes used in Table 1 below are listed together with the corresponding abbreviations:

A = Ala; F = Phe; G = Gly; D = Asp;
K = Lys; P = Pro; S = Ser; Y = Tyr.

TABLE 1

| Compound | Amino acid analysis | $F_0/F_\infty$ % | $k_{cat}/K_m$ $min^{-1}mM^{-1}$ |
| --- | --- | --- | --- |
| 6 ABz-FGY($NO_2$)D | F0.96; G1.04; Y(NO2) 1.00; D 1.00 | 0.3 | 1.4 |
| 7 ABz-AFGY($NO_2$)D | A 0.99; F 0.99; G 1.02; Y($NO_2$) 1.07; D 1.00 | 1.1 | 225 |
| 8 ABz-AAFGY($NO_2$)D | A 1.96; F 1.02; G 1.00; Y($NO_2$) 1.10; D 1.00 | 1.2 | 230000 |
| 9 ABz-AAAFGY($NO_2$)D | A 2.98; F 0.99; G 1.01; Y($NO_2$) 0.99; D 1.00 | 3.2 | 72000 |
| 10 ABz-AAAAFGY($NO_2$)D | A 3.96; F 0.97; G 0.97; Y($NO_2$) 1.05; D 1.00 | 3.4 | 320000 |
| 11 ABz-AAFSY($NO_2$)D | A 1.94; F 1.00; S 0.87; Y($NO_2$) 1.07; D 1.00 | 1.5 | 290000 |
| 12 ABz-APFGY($NO_2$)D | A 0.92; F 0.95; P 1.00; G 0.96; Y($NO_2$) 1.05; D 1.00 | 1.2 | 54000 |
| 13 ABz-SKSAAAAFGY($NO_2$)D | A 4.13; F 1.08; G 1.02; S 1.73; K 1.02; Y($NO_2$) 1.05; D 1.00 | 6.3 | 162000 |
| 14 ABz-DSGSKS-AAAAFGY($NO_2$)D | A 4.26; F 1.07; G 2.03; S 2.80; K 1.00; Y($NO_2$) 1.05 D 1.78 | 10.2 | 115000 |
| 15 ABz-SDSDSGSKS-AAAAFGY($NO_2$)D | A 4.19; F 1.16; G 2.03; S 5.0; K 1.06; Y($NO_2$) 1.11; D 3.00 | 18.5 | 135000 |

## Claims

1. A fluorogenic peptide exhibiting intramolecular quenching by resonance energy transfer and having the general formula

ABz-A-Q-$Y_1$ or $Y_2$-Q-A-ABz

wherein ABz designates the anthraniloyl donor chromophore and Q is a fluorescence quenching acceptor chromophore having an absorption spectrum overlapping the emission spectrum of the ABz donor chromophore, being a substituted amino acid residue of the general formula

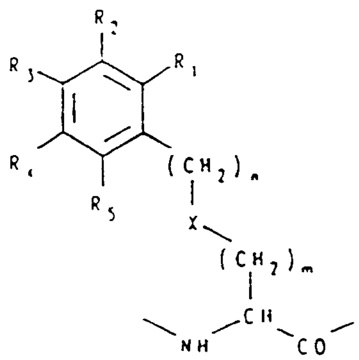

(I)

wherein $R_1$, $R_2$, $R_3$, $R_4$ and $R_5$ are independently selected from hydrogen, hydroxy, alkyl, alkoxy, nitro, amido, halogen or aralkyl, with the proviso that at least one of $R_1$ to $R_5$ is hydroxy and at least one is nitro,
wherein X is a spacing group which may be absent or selected from any combination of -CO-NH-, -CO-O-, -S-, -S-S-, -O-, -NH- or -$C_6H_4$-, and n and m are integers from 0 to 10,
A is a spacing entity selected from amino acid residues and peptide residues and may include a non-peptide molecular entity, being linked to the fluorescent donor chromophore ABz via an amide bond or ester bond,
$Y_1$ is OH, $NH_2$, an amino acid residue, a peptide residue or a C-terminal protective group,
$Y_2$ is H, an amino acid residue, a peptide residue or an N-terminal protective group.

2. A peptide according to claim 1 having the general formula

ABz-$A_1$-Tyr($NO_2$)-$Y_1$

wherein $A_1$ is an amino acid residue or a peptide residue linked to the group ABz via a peptide bond, and $Y_1$ is as defined above.

3. A peptide according to claim 1 or 2, wherein $Y_1$ is OH or an amino acid residue which is attachable to a solid phase or a peptide residue having a C-terminal amino acid residue which is attachable to a solid phase.

4. A peptide according to any of claims 1 to 3, wherein A or $A_1$ is an amino acid residue or a peptide residue comprising 2-25 amino acids.

5. A peptide according to any of claims 1 to 4, wherein A includes a mono- or oligosaccharide entity.

6. Substrate for determination of enzymatic activities comprising one or more fluorogenic peptides according to claims 1-5.

7. Process for the detection or determination of enzymatic activities in a sample,
**characterized** by incubating the sample with one or more substrates according to claim 6 under conditions favourable to enzymatic activities, determining and comparing the fluorescence emitted before, during and after the incubation.

## Patentansprüche

1. Fluorogenes Peptid mit intramolekularem Quenching durch Resonanzenergietransfer und mit der allgemeinen Formel

   ABz-A-Q-$Y_1$ oder $Y_2$-Q-A-ABz

   in der ABz einen Anthraniloyl-Donorchromophor bedeutet und Q ein Fluoreszenzquench-Akzeptorchromophor mit einem das Emmissionsspektrum des ABz-Donorchromophors überlappenden Absorptionsspektrum ist und einen substituierten Aminosäurerest der allgemeinen Formel

   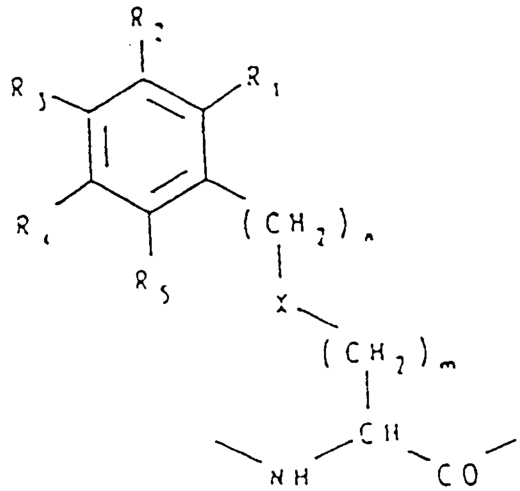
   (I)

   darstellt, in der $R_1$, $R_2$, $R_3$, $R_4$ und $R_5$ unabhängig voneinander aus Wasserstoff, Hydroxy, Alkyl, Alkoxy, Nitro, Amido, Halogen oder Aralkyl ausgewählt sind, mit der Maßgabe, daß mindestens eines der $R_1$ bis $R_5$ Hydroxy und mindestens eines Nitro ist, und in der X eine Spacergruppe ist, die fehlen kann oder aus jeder Kombination von -CO-NH-, -CO-O-, -S-, -S-S-, -O-, -NH- oder -$C_6H_4$-ausgewählt ist, und n und m ganze Zahlen von 0 bis 10 sind,
   A eine Spacereinheit ist, die aus Aminosäureresten und Peptidresten ausgewählt ist und eine nicht-peptidische Moleküleinheit einschließen kann, die mit dem fluoreszierenden Donorchromophor ABz über eine Amidbindung oder eine Esterbindung verknüpft ist,
   $Y_1$ OH, $NH_2$, ein Aminosäurerest, ein Peptidrest oder eine C-terminale Schutzgruppe ist, sowie
   $Y_2$ H, ein Aminosäurerest, ein Peptidrest oder eine N-terminale Schutzgruppe ist.

2. Peptid nach Anspruch 1 der allgemeinen Formel

   ABz-$A_1$-Tyr($NO_2$)-$Y_1$

   in der $A_1$ ein Aminosäurerest oder ein mit der Gruppe ABz über eine Peptidbindung verknüpfter Peptidrest und $Y_1$ wie oben definiert ist.

3. Peptid nach Anspruch 1 oder 2, bei dem $Y_1$ ein an eine feste Phase anheftbarer Aminosäurerest oder ein ein an an eine feste Phase anheftbaren C-terminalen Aminosäurerest aufweisender Peptidrest ist.

4. Peptid nach einem jeden der Ansprüche 1 bis 3, bei dem A oder $A_1$ ein Aminosäurerest oder ein 2 - 25 Aminosäuren umfassender Peptidrest ist.

5. Peptid nach einem jeden der Ansprüche 1 bis 4, bei dem A eine Mono- oder Oligosaccharid-Einheit umfaßt.

6. Substrat für die Bestimmung von enzymatischen Aktivitäten, enthaltend ein fluorogenes Peptid nach den Ansprüchen 1 bis 5 oder mehrere.

7. Verfahren für den Nachweis oder die Bestimmung von enzymatischen Aktivitäten in einer Probe, dadurch gekennzeichnet, daß man in die Probe mit einem Substrat gemäß Anspruch 6 oder mehreren unter für die enzymatischen Aktivitäten günstigen Bedingungen inkubiert und die vor, während und nach der Inkubation emittierte Fluoreszenz bestimmt und vergleicht.

## Revendications

1. Peptide fluorogène présentant une extinction intramoléculaire par le transfert d'énergie de résonance et ayant la formule générale :

   ABz-A-Q-$Y_1$ ou $Y_2$-Q-A-ABz

   où ABz désigne le chromophore donneur anthraniloyle et Q est un chromophore accepteur d'extinction de fluorescence ayant un spectre d'absorption chevauchant le spectre d'émission du chromophore donneur ABz, qui est un résidu d'acide aminé substitué de formule générale

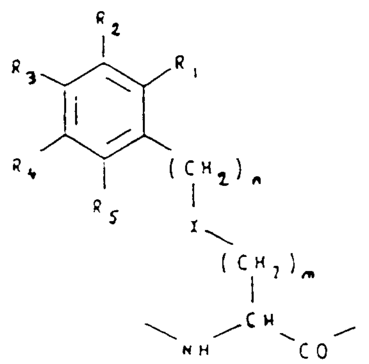

(I)

   où $R_1$, $R_2$, $R_3$, $R_4$ et $R_5$ sont indépendamment choisis parmi un atome d'hydrogène ou d'halogène ou un groupe hydroxy, alkyle, alcoxy, nitro, amido ou aralkyle, à condition qu'au moins un d'entre eux soit un groupe hydroxy et qu'au moins un soit un groupe nitro,
   où X est un groupe d'écartement qui peut être absent ou choisi parmi n'importe quelle combinaison suivante -CO-NH-, -CO-O-, -S-, -S-S-, -O-, -NH- ou -$C_6H_4$-, et n et m sont des entiers de 0 à 10,
   A est une entité d'écartement choisie parmi les résidus d'acide aminé et les résidus de peptide et peut renfermer une entité moléculaire non-peptidique, qui est liée au chromophore donneur fluorescent ABz par une liaison amide ou une liaison ester.
   $Y_1$ est un groupe OH, un groupe $NH_2$, un résidu d'acide aminé, un résidu de peptide ou un groupe protecteur C-terminal,
   $Y_2$ est l'atome H, un résidu d'acide aminé, un résidu de peptide ou un groupe protecteur N-terminal.

2. Peptide selon la revendication 1, de formule générale

   ABz-$A_1$-Tyr($NO_2$)-$Y_1$

   où $A_1$ est un résidu d'acide aminé ou un résidu de peptide lié au groupe ABz par une liaison peptidique et $Y_1$ est défini comme indiqué ci-dessus.

3. Peptide selon la revendication 1 ou 2, dans lequel $Y_1$ est un groupe OH ou un résidu d'acide aminé qui peut être attaché à une phase solide ou à un résidu de peptide ayant un résidu d'acide aminé C-terminal qui peut être attaché à une phase solide.

4. Peptide selon n'importe laquelle des revendications 1 à 3, dans lequel A ou $A_1$ est un résidu d'acide aminé ou un résidu de peptide comportant 2 à 25 acides aminés.

5. Peptide selon n'importe laquelle des revendications 1 à 4, dans lequel A renferme une entité mono- ou oligosaccharide.

**6.** Substrat pour la détermination d'activités enzymatiques comportant un ou plusieurs peptides fluorogènes selon les revendications 1 à 5.

**7.** Procédé pour la détection ou la détermination d'activités enzymatiques dans un échantillon, **caractérisé** par l'incubation de l'échantillon avec un ou plusieurs substrats selon la revendication 6 dans des conditions favorables aux activités enzymatiques, la détermination et la comparaison de la fluorescence émise avant, pendant et après l'incubation.

FIG. 1

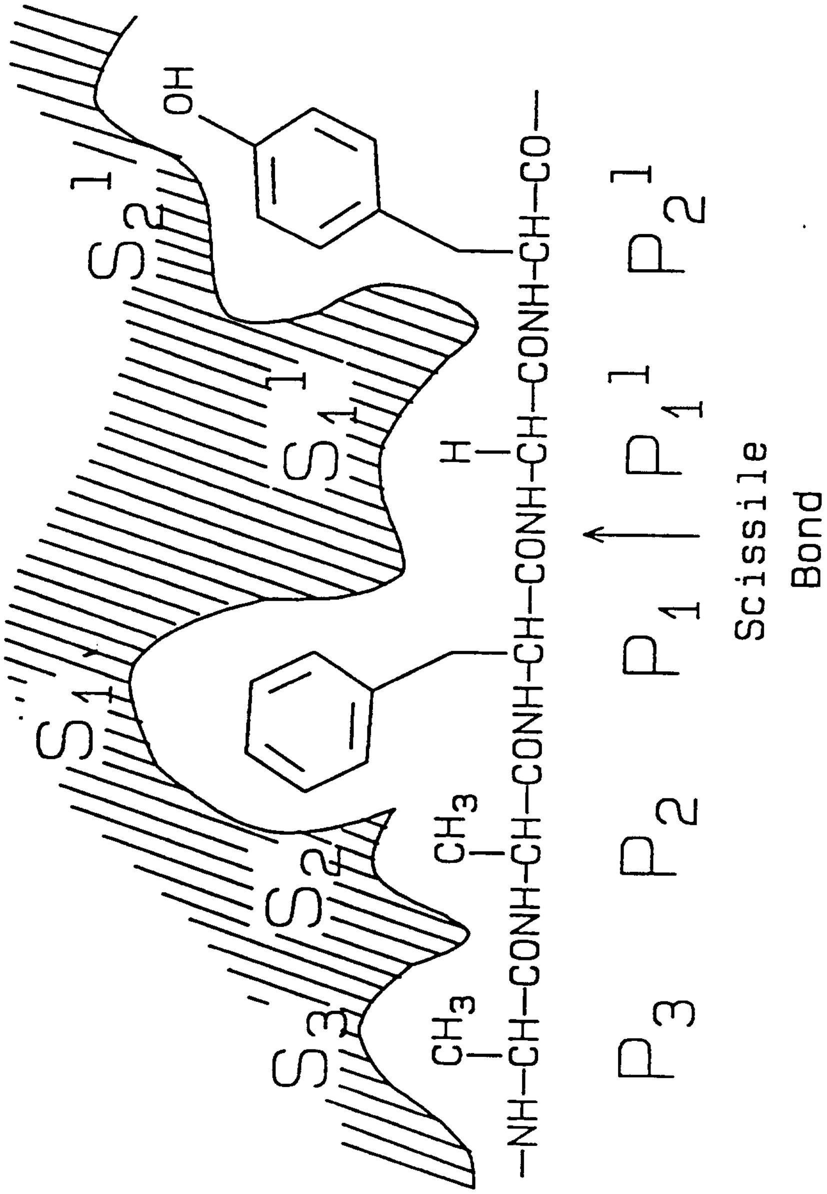

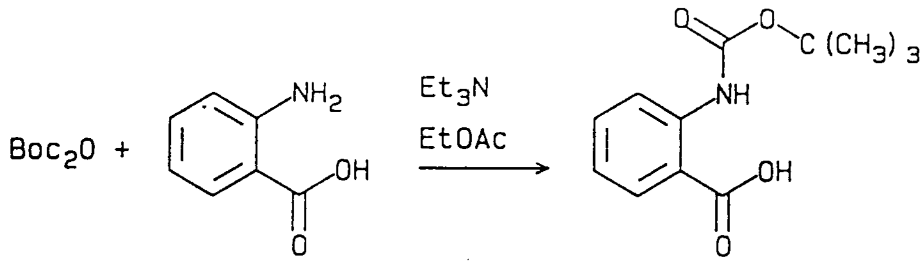


1 2

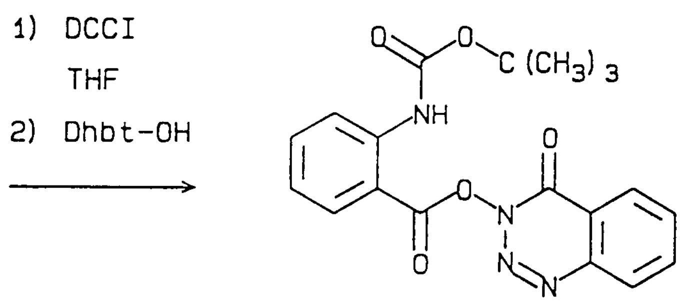


FIG. 2

3

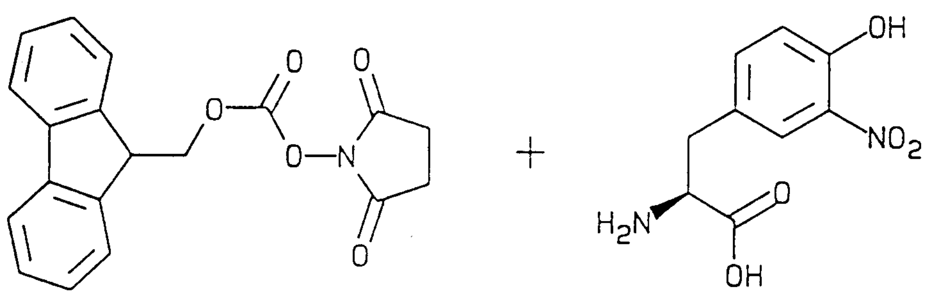

4

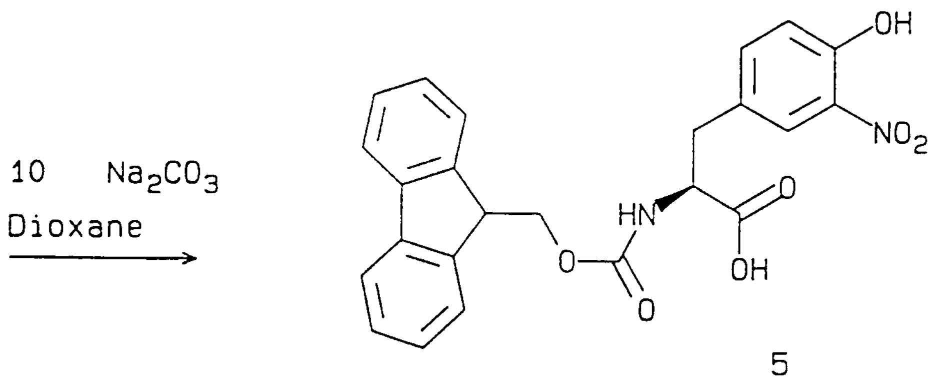


5

FIG. 3

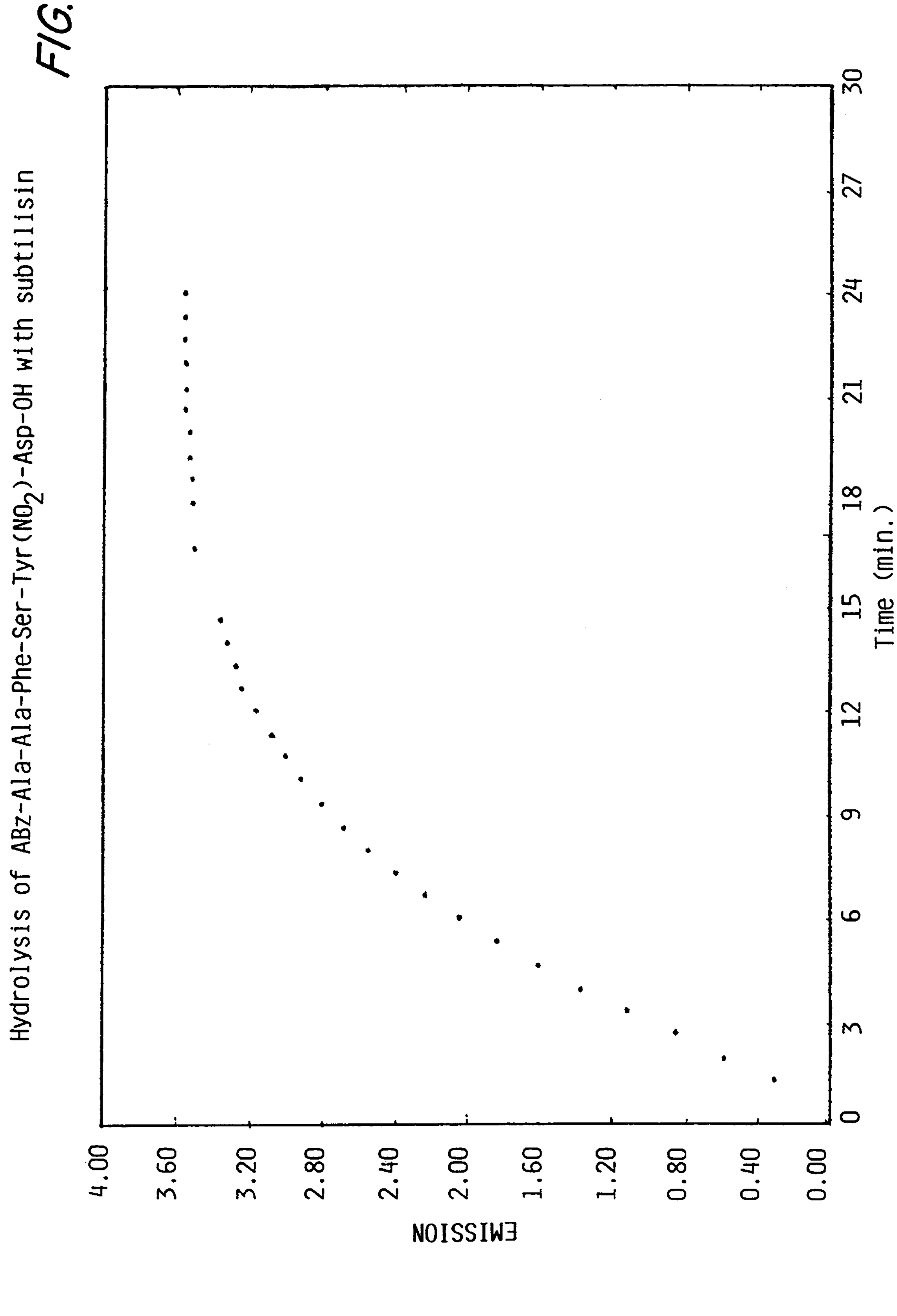
Hydrolysis of ABz-Ala-Ala-Phe-Ser-Tyr(NO2)-Asp-OH with subtilisin
EMISSION
0.00
0.40
0.80
1.20
1.60
2.00
2.40
2.80
3.20
3.60
4.00
0
3
6
9
12
15
18
21
24
27
30
Time (min.)